# EUROPÄISCHE PATENTSCHRIFT

(11) **EP 1 104 995 B1**
(45) Veröffentlichungstag und Bekanntmachung des Hinweises auf die Patenterteilung: **14.08.2002**
(21) Anmeldenummer: 99942756.0
(22) Anmeldetag: 29.06.1999
(51) Int. Cl.: A22B 5/00

(54) **VERFAHREN ZUR AUTOMATISCHEN KONTROLLE UND SELEKTION VON GEFLÜGELSCHLACHTTIERKÖRPERN**
METHOD FOR AUTOMATICALLY CONTROLLING AND SELECTING THE BODIES OF SLAUGHTERED POULTRY
PROCEDE DE CONTROLE ET DE SELECTION AUTOMATIQUES DE CORPS DE VOLAILLES ABATTUES

(30) Priorität: 20.08.1998 DE 19837804
(43) Veröffentlichungstag der Anmeldung: 13.06.2001
(73) Patentinhaber: CSB-System Software-Entwicklung & Unternehmensberatung AG, 52511 Geilenkirchen (DE)
(72) Erfinder: SCHIMITZEK, Peter, D-52511 Geilenkirchen (DE)
(74) Vertreter: Haussingen, Peter
(86) Internationale Anmeldenummer: DE9901938
(87) Internationale Veröffentlichungsnummer: WO0010397

(56) Entgegenhaltungen:
- DE-A- 4 408 604
- DE-A- 19 637 234
- DE-C- 19 619 099

## Beschreibung

Die Erfindung beschreibt ein Verfahren zur automatischen Kontrolle von Geflügelschlachttierkörpern, welche nach der Schlachtung mittels einer Fördereinrichtung an einer Kontrolleinrichtung vorbeitransportiert und anschließend abhängig vom Kontrollergebnis durch eine getriggerte optische Messung selektiert werden.

In modernen Schlachtlinien werden große Stückzahlen an Geflügel automatisch geschlachtet. Dadurch kommt der Sicherstellung der Qualitätskontrolle und der dadurch bedingten automatischen Selektion und Qualitätskontrolle eine wesentliche Bedeutung zu. Nur so kann sichergestellt werden, daß erstens keine krankhaften, beschädigten oder sonstwie anormalen Geflügelschlachttierkörper auf den Markt kommen und zweitens, daß bestimmte Produktqualitäten für bestimmte Vermarktungswege vorsortiert werden können.

Nach der Internationalen Patentklassifikation (IPC) wird die Verarbeitung von Geflügelschlachttierkörpern in der Klasse A22B 5/00 klassifiziert.

Nach der Druckschrift DE 196 19 099 C1 ist die berührungslose Bewertung von Geflügelschlachttierkörpern mittels Bildverarbeitung bekannt. Dabei wird eine optische Aufnahme des vollständigen Geflügelschlachttierkörpers photogrammetrisch ausgewertet und zur Parameterermittlung an Modelle angepaßt. Zusätzlich erfolgt eine Erfassung von Störstellenbereichen des Geflügelkörpers als andersfarbige Bildbereiche. Nachteilig bei dieser Lösung ist die aufwendige und kostenintensive photogrammetrische Verarbeitung zur Erfassung des Geflügelschlachttierkörpers bzw, dessen Körperteile. Die Konturerfassung birgt unnötige Fehlerrisiken und senkt die Zuverlässigkeit der Kontrolle der Geflügelschlachttierkörper.

In der Druckschrift DE 196 37 234 A1 wird ein Verfahren zur Online-Überprüfung der Farbreinheit von strukturierten oder nicht strukturierten Oberflächen, insbesondere zur Qualitätskontrolle beschrieben. Die Oberflächen werden mittels Video-Kamera erfasst und die erhaltenen elektronischen Signale mittels Computer ausgewertet. Gleichzeitig mit der Oberfläche werden Farbwerte einer oder mehrerer gemusterter Referenztafeln erfasst, wobei die optische Auflösung der Erfassungseinrichtung so niedrig eingestellt wird, dass die Struktur der Oberfläche nicht erfasst wird. Es werden integrale, bestimmte Farbwerte einer Fläche ermittelt. Abweichungen der spezifisch bestimmten Oberflächenfarbe von einem Soll/Referenzwert dienen beispielsweise der Qualitätseinstufung von Textilien oder auch von Fleisch. Die Verwendung von Referenztafeln, die Ermittlung von Bildpunkten gleicher Helligkeit jeweils für Rot, Grün und Blau erfordern einen entsprechend hohen Aufwand, um einen Farbvergleich zu realisieren. Mit diesem Verfahren ist keine einfache und kostengünstige Selektion von beschädigten oder anormalen Geflügelschlachttierkörpern möglich.

Die Aufgabe der Erfindung ist die Realisierung eines sehr einfachen und kostengünstigen Verfahrens zur automatischen Kontrolle und Selektion von Geflügelschlachttierkörpern.

Die Aufgabe wird durch die im Patentanspruch 1 genannten Merkmale gelöst. Bevorzugte Weiterbildungen ergeben sich aus den Unteransprüchen.

Das Wesen der Erfindung besteht in der Farbanalyse des von dem zu kontrollierenden Geflügelschlachttierkörper rückgestreuten, mittels eines Farblichtsensors empfangenen Farbmischlichts und die vorzugsweise in einem Rechner erfolgende anschließende Bewertung der Farbinformation zur bedingten Selektion. Durch die Verwendung eines diffusen Farbmischlichtes werden alle vorbestimmten sichtbaren Oberflächenregionen gleichzeitig erfaßt und über eine Farbanalyse bewertbar. Somit entfällt die weiterführende Bildbearbeitung einer optischen Aufnahme. Dabei macht sich die Erfindung die Erkenntnis zu Nutze, daß bei Geflügelschlachttierkörpern, welche im wesentlichen gleichartig sind, vordergründig die zuverlässige Selektion ganzer anormaler Körperregionen wie Brust, Flügel, Keule von Interesse ist.

In einer ersten Variante ist dieses Farbmischlicht optisch abbildungsfrei erzeugt mittels des objektivlosen Empfangs des diffus rückgestreuten Lichtes durch eine fest vorgegebene begrenzende Blende vor dem Geflügelschlachttierkörper. In einer zweiten Variante erfolgt eine abbildende Aufnahme des Geflügelschlachttierkörpers durch eine Farbkamera und die rechentechnische Bereitstellung des Farbbildes in dem Rechner, der über softwaremäßige Blenden den fest vorgegebenen Bereich einfach selektiert und nur die einzelnen Farbanteile jeweils akkumuliert, wodurch das Farbmischlicht somit einfach softwaremäßig erzeugt wird. Die Vorschaltung eines schmalbandigen Sperrfilters zur Unterdrückung eines monochromatischen Hintergrundes ist vorteilhaft.

Es ist prinzipiell denkbar, geeignete Schwellwertschalter je Farbinformation über logische Baugruppen zur Realisierung der Bewertung zu Verknüpfen. Der zur Bewertung und Steuerung der Selektion vorzugsweise benutzte Rechner beinhaltet lediglich die Bewertungsroutinen zur Selektion sowie in der zweiten Variante die Routinen zur Akkumulation innerhalb der Grenzen des vorgegebenen Bereichs sowie optional zu Kontrollzwecken zusätzlich einen Zwischenspeicherbereich für einen Farbbildbereich. Die Akkumulation der jeweiligen Farbanteile wird in der zweiten Variante innerhalb der - vorzugsweise rechteckigen - Grenzen des Farbbildbereich direkt beim Einlesen dessen aus der Farbkamera vorgenommen. Ein Microcontroller ist demnach prinzipiell als Rechner ausreichend. Insbesondere sind keine photogrammetrischen Bildbearbeitungsroutinen und deren weiterführende Leistungsparameter erforderlich.

Zur Farbanalyse des Farbmischlichtes werden vorzugsweise die von üblichen Farblichtsensoren bzw. Farbkameras benutzten Farbsignale (Rot, Grün, Blau) verwendet, die den spektralen Empfindlichkeiten des menschlichen Auges angenähert sind. Es ist jedoch auch eine Benutzung anderer Spektralbereiche vorstellbar. Die einzelnen Farbsignale des Farbmischlichtes bestimmen über ihre relativen Anteile die Farbe im Farbkreis sowie über ihre absolute Intensität die Farbsättigung und Helligkeit. Mit den Farbsignalen oder alternativ mit der Farbe, Farbsättigung und der Helligkeit läßt sich jeder erfaßbare Zustand des Mischlichtes einfach analytisch beschreiben, vorzugsweise vektoriell in einem mehrdimensionalen Raum. Insbesondere sind eindeutige Raumbereiche definierbar, die den Selektionskriterien entsprechen. Derartige Selektionskriterien sind bsw. fehlende Körperteile, Verbrühungen, Rötungen, fehlende Haut, hervorstehende Knochen, usw.
Die Gesamtheit aller Raumbereiche sollte vorzugsweise den gesamten Raum ohne Überlappungen ausfüllen. Liegen Parameter des analysierten Farbmischlicht in einem Raumbereich dieser Selektionskriterien, wird die zugeordnete Selektion, bspw. Kennzeichnung, Aussonderung, Teilverarbeitung, aktiviert.

Es ist des weiteren vorstellbar, daß je Messung und je Farbe ein Farbabgleich über das integrale Mittel einer Vielzahl von erfaßten Geflügelschlachttierkörpern - Weißabgleich - und vorzugsweise alternierend des bloßen Hintergrundes - Schwarzabgleich - vorgenommen wird, indem dieser integrale Mittelwert jeweils vor der Bewertung als Normierungsgröße - durch welche geteilt wird - bzw. als Offset - um welche der Meßwert zuvor vermindert wird - behandelt wird. Dadurch wirken sich Schwankungen der Lichtverhältnisse bzw. der Sensorkennwerte nicht wesentlich auf das Bewertungsergebnis aus. Durch die Normierung erfolgt eine Projektion des Bewertungsraumes auf eine zugeordnete Bewertungsebene, vorzugsweise Farbe und Farbsättigung.

Es besteht ebenfalls die Möglichkeit unter Verwendung einer speziell angepaßten Blende eine Mehrzahl von Farblichtsensoren einzusetzen, die jeweils einem bestimmten zu kontrollierenden Körperteil oder einer Körperpartie des Schlachttieres zugeordnet sind (Brust, linke Keule, rechter Flügel). Bei Einsatz einer Farbkamera kann die Selektion der zu kontrollierenden unterschiedlichen Körperteile und Körperpartien über mehrere softwaremäßige Blenden erfolgen.

Günstigerweise könnte die Kontrollvorrichtung im Bedarfsfall auch aus zwei gleichartigen Anordnungen von Farblichtsensoren beinhalten, die sich jeweils gegenüberliegen und so gleichzeitig das rückgestreute Farbmischlicht der Vorder- und Rückseite des Geflügelschlachttierkörpers kontrollieren.

Eine weitere mögliche Ausbildung der Kontrollvorrichtung könnte die zusätzliche rückseitige Anordnung eines optischen Spiegels gegenüber dem Farblichtsensor beinhalten, sodaß gleichzeitig das rückgestreute Farbmischlicht von zwei sich gegenüberliegenden Seiten des Geflügelschlachttierkörpers empfangen und kontrolliert werden kann.

Die Erfindung wird als Ausführungsbeispiel näher an
Fig. 1 als Variante 1 mit optischen Blenden
Fig. 2 als Variante 2 mit Softwareblenden
erläutert.

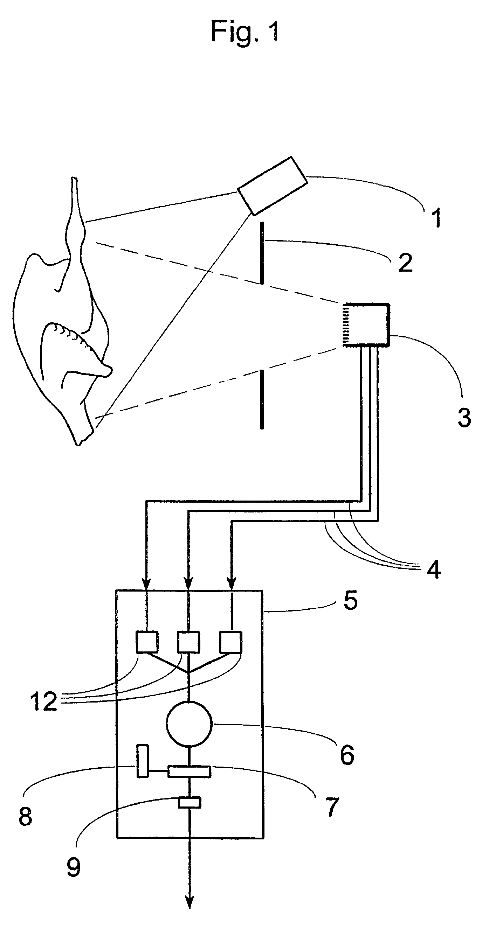

Nach Fig. 1 besteht die Anordnung zur automatischen Kontrolle und Selektion von Geflügelschlachttierkörpern mit optischen Blenden aus einer weißes Licht emittierenden Beleuchtungseinrichtung 1, einer Blende 2 vor dem Geflügelschlachttierkörper, die den sichtbaren Umriß eng umgrenzt, einem objektivlosen Farblichtsensor 3, der positionsgetriggert das vom Geflügelschlachttierkörper rückgestreute diffuse Farbmischlicht empfängt und dessen Farbinformationen über eine Leitung 4 zu einem Rechner 5 übertragen werden.

Im Rechner 5 erfolgt eine Farbanalyse 6 des rückgestreuten Farbmischlichts basierend auf den benutzten Farbsignalen. Nach interner Bewertung 7 der Farbanalyse gemäß Selektionskriterien 8 wird ein Steuersignal 9 entsprechend der jeweils dem Geflügelschlachttierkörper zugeordneten Selektionsart zur Ansteuerung von Selektionseinrichtungen im technologischen Prozeß ausgegeben.

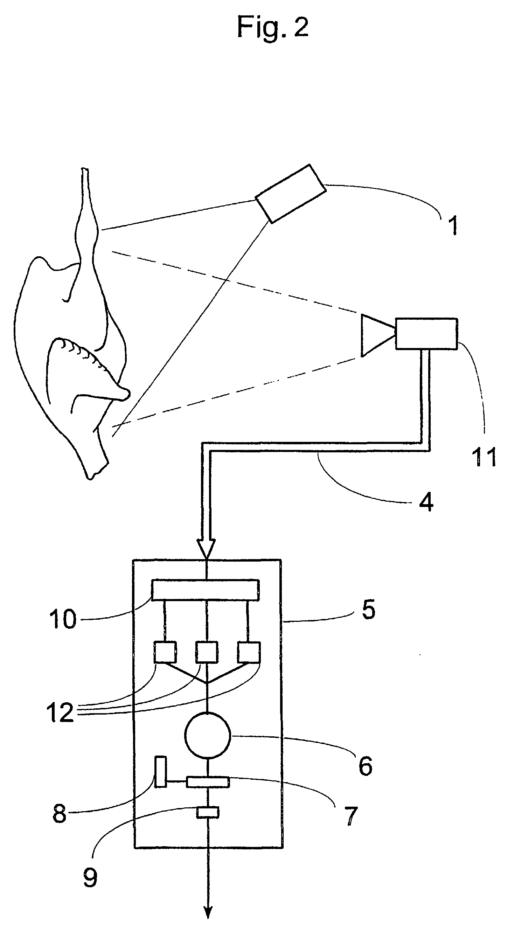

Nach Fig. 2 besteht eine mögliche Variante der Anordnung zur automatischen Kontrolle und Selektion von Geflügelschlachttierkörpern mit softwaremäßigen Blenden 10 aus einer weißes Licht emittierenden Beleuchtungseinrichtung 1, einer Farbkamera 11 mit Farbsensorarray, deren Farbbild über eine Leitung 4 zu einem Rechner 5 übertragen wird. Ein fest vorgegebener Bereich des Farbbildes wird beim Einlesen in den Rechner 5 mittels einer softwaremäßiger Blende 10 selektiert, und je Farbinformation in einem Farbinformationsspeicher 12 akkumuliert. Dies kann bei zeilenweiser Übertragung des Farbbildes vorteilhaft erfolgen, wenn über geeignete Zähler ein rechteckiger Bereich als sofwaremäßige Blende 10 selektiv bezüglich Zeilen/Spalten gefiltert wird. Im Rechner 5 erfolgt eine Farbanalyse 6 des repräsentierten Farbmischlichts basierend auf den Farbinformationsspeichern 12. Nach interner Bewertung 7 der Farbanalyse 6 gemäß der Selektionskriterien 8 wird ein Steuersignal 9 entsprechend der jeweils dem Geflügelschlachttierkörper zugeordneten Selektionsart zur Ansteuerung von Selektionseinrichtungen im technologischen Prozeß ausgegeben.

### Verwendete Bezugszeichen

- 1: Beleuchtungseinrichtung
- 2: Blende
- 3: Farblichtsensor
- 4: Leitung
- 5: Rechner
- 6: Farbanalyse
- 7: Bewertung
- 8: Selektionskriterien
- 9: Steuersignal
- 10: softwaremäßige Blende
- 11: Farbkamera
- 12: Farbinformationsspeicher

## Patentansprüche

1. Verfahren zur automatischen Kontrolle und Selektion von Geflügelschlachttierkörpern mittels Farbanalyse des von den sichtbaren Oberflächenteilen rückgestreuten Lichts, **dadurch gekennzeichnet,**
**daß** dieses die räumliche Kontur eliminierende diffuse Farbmischlicht meßtechnisch erfaßt wird und dessen Meßwert als Integrationswert über die Gesamtheit der sichtbaren Oberflächenteile zur Selektion benutzt wird.

2. Verfahren zur automatischen Kontrolle und Selektion von Geflügelschlachttierkörpern nach Anspruch 1, **dadurch gekennzeichnet,**
**daß** das durch eine fest vorgegebene Blende (2) dringende Farbmischlicht objektivlos von einem Farblichtsensor (3) aufgenommen wird.

3. Verfahren zur automatischen Kontrolle und Selektion von Geflügelschlachttierkörpern nach Anspruch 1, **dadurch gekennzeichnet,**
**daß** das von einer Farbkamera (11) erfaßte Farbbild über fest vorgegebenen softwareförmigen Blenden (10) zu einem Farbmischlicht im Farbinformationsspeicher (12) akkumuliert wird.

4. Verfahren zur automatischen Kontrolle und Selektion von Geflügelschlachttierkörpern nach einem der Ansprüche 1 bis 3, **dadurch gekennzeichnet,**
**daß** je Messung und Farbe ein integraler Abgleich über eine Vielzahl von Messungen vorgeschaltet ist.

5. Verfahren zur automatischen Kontrolle und Selektion von Geflügelschlachttierkörpern nach einem der Ansprüche 1 bis 4, **dadurch gekennzeichnet,**
**daß** an mehreren signifikanten, unterschiedlichen Körperpartien eine Messung erfolgt.

6. Verfahren zur automatischen Kontrolle und Selektion von Geflügelschlachttierkörpern nach einem der Ansprüche 1 bis 5, **dadurch gekennzeichnet,**
**daß** die Kontrollvorrichtung aus zwei gleichen Anordnungen mit Farblichtsensor (3) bzw. Farbkamera (11) besteht, die sich jeweils gegenüberliegen und so gleichzeitig Vorderund Rückseite des Geflügelschlachttierkörpers kontrollieren.

7. Verfahren zur automatischen Kontrolle und Selektion von Geflügelschlachttierkörpern nach einem der Ansprüche 1 bis 5, **dadurch gekennzeichnet,**
**daß** die Kontrollvorrichtung die zusätzliche rückseitige Anordnung eines optischen Spiegels gegenüber dem Farblichtsensor aufweist, sodaß gleichzeitig das rückgestreute Farbmischlicht von zwei sich gegenüberliegenden Seiten des Geflügelschlachttierkörpers empfangen und kontrolliert werden kann.

## Claims

1. Process of automated quality control and selection of slaughtered poultry carcasses by means of colour analysis of the light which is back-scattered from the visible surface parts, **characterised in that** this diffused colour mixing light which eliminates the spatial contour is detected using measuring technology and its measured value is used as an integration value over the entirety of the visible surface parts for the purpose of selection.

2. Process of automated quality control and selection of slaughtered poultry carcasses according to claim 1, **characterised in that** the colour mixing light which passes through a fixedly predetermined aperture (2) is recorded without a lens by a colour light sensor (3).

3. Process of automated quality control and selection of slaughtered poultry carcasses according to claim 1, **characterised in that** the colour image recorded by a colour camera (11) is accumulated by way of fixedly predetermined software apertures (10) to form a colour mixing light in the colour information memory (12).

4. Process of automated quality control and selection of slaughtered poultry carcasses according to any one of claims 1 to 3, **characterised in that** for each measurement and colour, an integral comparison is incorporated over a plurality of measurements.

5. Process of automated quality control and selection of slaughtered poultry carcasses according to any one of claims I to 4, **characterised in that** a measurement is taken on several significant, different carcass portions.

6. Process of automated quality control and selection of slaughtered poultry carcasses according to any one of claims I to 5, **characterised in that** the inspection device consists of two identical arrangements having a colour light sensor (3) or colour camera (11) which lie opposite one another in each case and in this way inspect the front side and rear side of the slaughtered poultry carcass simultaneously.

7. Process of automated quality control and selection of slaughtered poultry carcasses according to any one of claims 1 to 5, **characterised in that** the inspection device comprises the additional rear-side arrangement of an optical mirror opposite the colour light sensor, so that the back-scattered colour mixing light of two mutually opposite sides of the slaughtered poultry carcass can be received and inspected simultaneously.

## Revendications

1. Procédé de contrôle automatique et de sélection de corps de volailles abattues, par analyse de la couleur de la lumière renvoyée par les parties superficielles visibles des volailles,
**caractérisé en ce que**
cette lumière à couleurs mélangées, diffuse et éliminant le contour spatial est saisie par une technique de mesure et la valeur mesurée est utilisée comme valeur d'intégration concernant l'ensemble des parties superficielles visibles, pour effectuer une sélection.

2. Procédé selon la revendication 1,
**caractérisé en ce que**
la lumière à couleurs mélangées, traversant un écran (2) déterminé fixement à l'avance, est reçue sans objectif par un détecteur de lumière à couleurs mélangées (3).

3. Procédé selon la revendication 1,
**caractérisé en ce que**
l'image en couleurs prise par une caméra à couleurs (11), à travers des écrans (10) déterminés fixement à l'avance au niveau du programme, est stockée pour donner une lumière à couleurs mélangées dans la mémoire des informations de couleur (12).

4. Procédé selon l'une quelconque des revendications 1 à 3,
**caractérisé en ce que**
pour chaque mesure et chaque couleur, il est effectué préalablement une égalisation intégrale sur un certain nombre de mesures.

5. Procédé selon l'une quelconque des revendications 1 à 4,
**caractérisé en ce qu'**
on effectue une mesure sur plusieurs parties significatives, différentes, du corps.

6. Procédé selon l'une quelconque des revendications 1 à 5,
**caractérisé en ce que**
le dispositif de contrôle est composé de deux systèmes identiques comprenant chacun un détecteur de lumière de couleur (3) ou une caméra couleurs (11), qui sont disposés à l'opposé l'un de l'autre et contrôlent ainsi en même temps l'avant et l'arrière du corps de volaille abattue.

7. Procédé selon l'une quelconque des revendications 1 à 5,
**caractérisé en ce que**
le dispositif de contrôle comprend en plus un système situé vers l'arrière et comportant un miroir optique situé en face du détecteur de lumière colorée, de sorte qu'il est possible de recevoir et de contrôler en même temps la lumière à couleurs mélangées renvoyée par deux côtés opposés du corps de volaille abattue.
